# EUROPEAN PATENT APPLICATION

(11) **EP 3 556 473 A1**
(43) Date of publication of application: **23.10.2019**
(21) Application number: 18167444.1
(22) Date of filing: 16.04.2018
(51) Int. Cl.: B05B 9/01, B05B 7/02

(54) **SPRAY DEPOSITION SYSTEM AND ITS USE FOR TREATING A LIVING BEING**

(71) Applicant: RenovaCare Sciences Corp., New York, NY 10022 (US)
(72) Inventor: Schubert, Frank, 10242 Berlin (DE); Bhogal, Jatinder S., Vancouver, BC V6B 1P1 (CA); Bold, Thomas, 12049 Berlin (DE)
(74) Representative: Pfenning, Meinig & Partner mbB

(57) **Abstract**

The present invention provides a spray deposition system which comprises a syringe attachment device having a specific adaptor. The adaptor has at least three openings being in fluid connection with each other, wherein at least one first opening of the adaptor is connectable to a needle in a liquid-tight manner, at least one second opening of the adaptor is in fluid connection with surrounding air and at least one third opening of the adaptor is connected to a tube configured to deliver an air flow in a reversible and air-tight manner. Thereby, a connection to a needle is possible in a reversible and liquid-tight manner without having to pierce the tube of the system like in prior art solutions. This provokes that no tube material may clog the needle and no air may leak from the tube. Furthermore, an attachment of the needle to the spray deposition system may be performed faster and safer and alignment of the needle is facilitated. Finally, dulled needles may be employed which ensure a more homogeneous deposition of liquid from the needle.

## Description

The present invention provides a spray deposition system which comprises a syringe attachment device having a specific adaptor. The adaptor has at least three openings being in fluid connection with each other, wherein at least one first opening of the adaptor is connectable to a needle in a liquid-tight manner, at least one second opening of the adaptor is in fluid connection with surrounding air and at least one third opening of the adaptor is connected to a tube configured to deliver an air flow in a reversible and air-tight manner. Thereby, a connection to a needle is possible in a reversible and liquid-tight manner without having to pierce the tube of the system like in prior art solutions. This provokes that no tube material may clog the needle and no air may leak from the tube. Furthermore, an attachment of the needle to the spray deposition system may be performed faster and safer and alignment of the needle is facilitated. Finally, dulled needles may be employed which ensure a more homogeneous deposition of liquid from the needle.

Spraying of cells may be of interest for the distribution of cell suspensions on a specific surface, such as, for example, a tissue wound. The spraying of cells is used, for example, in surgery in order to regenerate traumatized tissue. In many scenarios, it is beneficial or critical that the deposition system for the cells be sterile. Although the cell deposition or spraying can be done manually, an electrically operated and electronically controlled device can allow for controlled application of the cells onto the surface. Such devices can have a motor drive, a battery and other components. It can be difficult to sterilize portions of these devices since the sterilization process can potentially harm or destroy these components.

Furthermore, prior art solutions of spray deposition systems suffer the disadvantage that a connection of a needle to the tube of the system which is configured to deliver an air flow necessitates puncturing said tube with the needle. This effectuates that tube material may enter the interior of the needle which may result in clogging of the needle. In addition, there is a risk that air passing the tube may leak from the tube at the site of punctuation resulting in an inefficient and/or uncontrolled air stream. Furthermore, in prior art solutions, an attachment of the needle to the spray deposition system may consume a certain amount of time until the needle is pierced through the tube and the piercing operation is connected to a risk for injuries. Additionally, a proper alignment of the needle in the spray deposition system may be time-consuming and cumbersome. What is more, the piercing of the needle through the tube may only be performed with needles having a certain sharpness and inherent asymmetry at the tip. These needles are known to provide a less homogeneous deposition of liquid from the needle than needles with a dulled tip.

Starting herefrom, it was the object of the present application to provide a spray deposition system which does not have the above disadvantages.

The object is solved by the spray deposition according to claim 1 and the spray deposition system for the use according to claim 15. The dependent claims illustrate advantageous embodiments.

According to the invention, a spray deposition system is provided, comprising:
a) a syringe attachment device having a base with a coupling portion and a top portion, the syringe attachment device comprising:
   i) a syringe channel configured to receive at least parts of a syringe; and
   ii) a tube configured to deliver an air flow;
b) a handheld device having an upper surface and including a movable slide disposed on the upper surface, wherein the handheld device is configured to be coupled to the coupling portion of the syringe attachment device in an assembled position of the system and wherein the handheld device is configured to control delivery of a treatment solution from the syringe;
characterized in that the syringe attachment device comprises an adaptor having at least three openings being in fluid connection with each other, wherein at least one first opening of the adaptor is connectable to a needle in a liquid-tight manner, at least one second opening of the adaptor is in fluid connection with surrounding air and at least one third opening of the adaptor is (fluidly) connected to the tube in a reversible and air-tight manner.

According to the invention, the term "fluid connection" has the meaning that a fluid (e.g. a liquid and/or a gas) can flow through the connection. For example, the meaning of the at least three openings of the adaptor being in fluid connection with each other is that a fluid (e.g. a liquid and/or a gas) can flow from each of the at least three openings to any other of the at least three openings of the adaptor and *vice versa.* By the same token, the meaning of at least one second opening of the adaptor being in fluid connection with surrounding air is that a fluid (e.g. a liquid and/or a gas) can flow from said second opening of the adaptor to the surrounding air and *vice versa.*

The advantage of the inventive spray deposition device is that the adaptor effectuates that a connection to a needle is possible in a liquid-tight manner without having to pierce the tube of the system like in prior art solutions. This provides that no tube material may clog the needle and no air may leak from the tube. Furthermore, an attachment of the needle to the spray deposition system may be performed faster and without a risk of injuries. Moreover, an alignment of the needle is facilitated because the shape of the adapter provides a guidance path for the needle and the occurrence of a leakage by a piercing through the whole tube (i.e. a piercing into and out of the tube) is not possible. Finally, since no piercing through a tube wall is necessary, dulled needles may be employed which provide a more homogeneous deposition of liquid from the needle and thus a more homogeneous spray deposition with the spray deposition system.

In one preferred embodiment, the at least one first opening of the adaptor is connectable to a needle in a liquid-tight manner and reversible manner.

The spray deposition system may be characterized in that the at least one first and at least one second opening of the adaptor are fluidly connected by a first fluid channel.

According to the invention, the term "fluidly connected" has the meaning that a fluid (e.g. a liquid and/or a gas) can flow through the connection. For example, the meaning of the at least one first and the at least one second opening of the adaptor being fluidly connected by a first fluid channel is that a fluid (e.g. a liquid and/or a gas) can flow from the at least one first opening to the at least one second opening of the adaptor via the first fluid channel and *vice versa.*

The first fluid channel preferably has an essentially straight form. An essentially straight form causes that the insertion and alignment of a needle in said first channel is easier compared to other geometries of the fluid channel.

The first fluid channel may have a length sufficiently large to accommodate a needle, preferably in a manner that the tip of the needle becomes located essentially at the same pitch like the at least one third opening. This is connected to the advantage that the tip of the needle does not protrude out of the adaptor and a risk of an injury by the needle tip is minimized.

The length of the first fluid channel may be 20 to 40 mm, preferably 22 to 36 mm, more preferably 24 to 32 mm, most preferably 25 to 29 mm, especially 26 to 28 mm. These lengths are advantageous because the first fluid channel is long enough to accommodate a needle and is sufficiently compact for a low weight and low production costs.

The first fluid channel may have a first section for liquid-tightly, optionally also reversibly, connecting a needle adaptor suitable for connecting a needle to a syringe, wherein the first section extends from the at least one first opening of the adaptor towards the at least one second opening of the adaptor, wherein the first section optionally has a diameter of 0.5 to 8 mm, preferably 1 to 7 mm, more preferably 2 to 6 mm, most preferably 2.5 to 5.5 mm, and/or a length of 10 to 20 mm, preferably 12 to 18 mm, more preferably 14 to 16 mm.

The first fluid channel may have a second section for accommodating a needle, wherein the second section is located away from the at least one first opening of the adaptor, wherein the second section optionally has a diameter of 0.2 to 3 mm, preferably 0.5 to 2.5 mm, more preferably 1.0 to 2.0 mm, most preferably 1.4 to 1.6 mm, and/or a length of 5 to 15 mm, preferably 6 to 14, more preferably 8 to 12, most preferably 9 to 11 mm. In a preferred embodiment, the diameter of the second section of the first fluid channel is smaller than the diameter of the first section of the first fluid channel to which the needle adaptor is connected. This provokes that the needle adaptor may not be pushed into the second section of the first fluid channel and thus the needle may not accidently be pushed through the first fluid channel. Thus, the risk of injuries is minimized.

The first fluid channel may have a third section for spray deposition of contents of a syringe, wherein the third section is located at the at least one second opening, wherein the third section preferably has a conical form (wherein the cone preferably opens toward the surrounding air), optionally with a maximum diameter of at least 2 mm, preferably at least 3 mm, more preferably at least 4 mm, most preferably at least 5 mm, and/or a length of 0.5 to 4 mm, preferably 1 to 2 mm, more preferably 1.5 to 2.5 mm. The conical form of the third section has the advantage that a defined spray cone may be formed which allows a more defined deposition of the sprayed substances. Additionally, adjustment of the cone opening diameter and/or the position of the needle along the first fluid channel (especially along the third section of the first fluid channel), allows an adjustment of the spray cone. This is beneficial if e.g. spray distance has to stay constant and skin wounds of different lateral extensions have to be treated.

The first fluid channel may have a needle located therein, preferably a needle having a dulled tip. A needle with a dulled tip has the advantage that the exit of the substances to be sprayed from the needle tip is more regular and consistent. This provokes a more homogeneous distribution of the sprayed substances on the target. The dulled tip of the needle may have a trumpet form. A trumpet form is connected to the advantage that - like a cone form of the third section of the fluid channel - the spray cone may be adjusted and fine-tuned to certain needs.

The spray deposition system may be characterized in that the at least one third opening is fluidly connected to the at least one first and second opening of the adaptor by a second fluid channel.

The second fluid channel preferably has an essentially straight form. The advantage of having an essentially straight form is that gas may travel through the second fluid channel in the shortest possible distance and with minimal turbulence.

The length of the second fluid channel may be 4 to 40 mm, preferably 6 to 28 mm, more preferably 8 to 16 mm, most preferably 10 to 14 mm, especially 11 to 13 mm.

The second fluid channel may have a section for accommodating the tube configured to deliver an air flow, wherein said section is located at the at least one third opening, wherein said section preferably has a diameter of 1 to 5 mm, preferably 1.5 to 4.5 mm, more preferably 2 to 4 mm, most preferably 2.5 to 3.5 mm, and/or a shorter length than the second fluid channel, optionally a length of 1 to 30 mm, preferably 2 to 25 mm, more preferably 4 to 20 mm, most preferably 6 to 15 mm, especially 8 to 10 mm.

In a preferred embodiment, the second fluid channel fluidly connects the first fluid channel in an angle of 10° to 90°, preferably in an angle of 20° to 90°, more preferably in an angle of 30° to 90°, even more preferably in an angle of 40° to 90°, particularly preferred in an angle of 50° to 90°, especially in an angle of 60° to 90°. Furthermore, it is preferred if the second fluid channel fluidly connects essentially perpendicular to the first fluid channel, wherein the connection point preferably has essentially the form of a T.The term "essentially" in this context means that the connection does not have to be exactly established at an angle of 90°, but may well be established at an angle of 70° to 90°, preferably at an angle of 80° to 90°, more preferably at an a angle of 85° to 90°.In a particularly preferred embodiment, the second fluid channel fluidly connects to the first fluid channel at a section of the first fluid channel at which a part of the needle becomes located which is closer an adaptor of the needle than to a tip of the needle. For example, this may be the second section of the first fluid channel, preferably a part of the second section of the first fluid channel which is closer to the first section of the first fluid channel than to the third section of the first fluid channel. The advantage of this embodiment is that the air streaming through the second fluid channel enters the first fluid channel at a position at which the needle base (i.e. the part of the needle close to the needle adaptor) and not the needle tip is located. This provides that liquid exiting the needle tip is prevented from penetrating the second fluid channel and the tube configured to deliver an air flow. Therefore, a contamination of said channel and said tube with contents of the syringe is obviated.

The first opening of the adaptor may be suitable for reversibly and liquid-tightly connecting to a Luer-lock connector or is connected thereto, preferably a Luer-lock connector of a needle, more preferably a Luer-lock connector of a PrecisionGlide Needle 30G1/2 from Becton & Dickinson. This has the advantage that the reversible and liquid-tight connection may be easily established.

In a preferred embodiment, the at least one first opening of the adaptor is connected to a needle. This embodiment has the advantage that no needle has to be assembled with the spray deposition system, because it is already equipped with a needle. In other words, for spraying a content of a syringe with the spray deposition system, only the syringe has to be connected to the system. Thus, this embodiment is characterized by an easier, faster and more convenient handling.

The adaptor of the spray deposition system may be reversibly connected to the syringe attachment device of the spray deposition device. The advantage is that the adaptor may be easily exchanged and easily sterilized.

The adaptor of the spray deposition system may be sterile.

The adaptor of the spray deposition system may comprise or consist of a plastics material, preferably a plastics material selected from the group consisting of polyurethane, polycarbonate, polyvinylchloride, acrylonitrile-butadiene-styrene, more preferably polyurethane SG 95, optionally chemically bonded with cyclohexanone and/or Loctite 4305. The advantage of said materials is that they are not expensive, are robust and resist sterilization procedures.

The syringe channel of the syringe attachment device of the spray deposition system and/or the tube of the syringe attachment device of the spray deposition system may (also) comprise or consist of a plastics material, preferably a plastics material selected from the group consisting of polyurethane, polycarbonate, polyvinylchloride, acrylonitrile-butadiene-styrene, more preferably polyurethane SG 95, optionally chemically bonded with cyclohexanone and/or Loctite 4305.

Furthermore, the handheld device of the spray deposition system may comprise or consist of a plastics material, preferably a plastics material selected from the group consisting of polyurethane, polycarbonate, polyvinylchloride, acrylonitrile-butadiene-styrene, more preferably polyurethane SG 95, optionally chemically bonded with cyclohexanone and/or Loctite 4305.

The syringe attachment device of the spray deposition system may comprise a coupling portion including a bottom surface configured to couple with a top portion of the handheld device in the assembled portion.

The syringe attachment device may comprise at least one feature on the coupling portion to engage a corresponding feature on an upper surface of the handheld device.

Preferably, the syringe attachment device comprises an air filter coupled to the tube. The air filter may sterilize air passing through the tube.

Optionally, the syringe attachment device comprises an airflow indicator coupled to the tube.

The syringe attachment device may comprise a needle having a taper fitting.

The handheld device of the spray deposition system may comprise a movable slide including a plunger driver configured to engage a plunger of a syringe disposed in the syringe channel, wherein the plunger is preferably configured to be electronically positionable or manually positionable by a user of the spray deposition system.

The handheld device of the spray deposition system may comprise a movable slide being controlled by a threaded shaft, wherein a coupler is preferably positioned between and configured for engagement with the movable slide and the threaded shaft, and movement of the coupler on the threaded shaft preferably corresponds to movement of the slide on the handheld device.

Optionally, the handheld device of the spray deposition system may comprise a switch configured to electronically control a movable slide.

The handheld device of the spray deposition system may comprise a battery compartment, wherein an electrical contact of the battery compartment is preferably electrically coupled to the electronically movable slide.

Optionally, the handheld device of the spray deposition system may comprise spray volume indicia.

The handheld device of the spray deposition system may comprise a first mark and a sterile cover includes a second mark, and the first and second marks are configured for alignment to determine placement of the sterile cover around at least a portion of the handheld device, in the assembled position.

The handheld device may be inserted into a sterile cover and the sterile cover encloses the handheld device in the assembled position.

Optionally, the handheld device of the spray deposition system may be reusable and/or the syringe attachment device may be disposable after use.

The spray deposition system may further comprise a sterile cover.

The sterile cover may preferably be coupled to the syringe attachment device, optionally between a top plate and a bottom plate of the syringe attachment device.

The sterile cover may be coupled to the syringe attachment device, wherein a top portion of the syringe attachment device is exposed and the sterile cover surrounds the coupling portion of the syringe attachment device.

The sterile cover may be welded to an outer perimeter of the base of the syringe attachment device. Optionally, the sterile cover may be bonded with an adhesive to an outer perimeter of the base of the syringe attachment device.

The sterile cover may have a cavity in which the handheld device is at least partially located in the assembled position.

In a preferred embodiment, the sterile cover encloses the handheld device in the assembled position.

The sterile cover may have a first end which is closed and a second end which is open, wherein the second end is preferably sealable after the handheld device is inserted into a sterile cover.

The sterile cover may be a sheet open on all sides, wherein the handheld device is covered by the sheet in an assembled position, and the sheet can be closed such that a seal is created between an interior of the sterile cover, containing the handheld device, and exterior of the sterile cover.

The spray deposition system may be characterized in that the coupling portion of the syringe attachment device is coupled to the handheld device in a reversible manner or in an irreversible manner, wherein preferably, the coupling portion of the syringe attachment device is monolithic to the handheld device. The advantage of the coupling portion of the syringe attachment device being monolithic to the handheld device is that the stability of the spray deposition is higher because no (reversible or irreversible) connection has to be established between the coupling portion and the handheld device to establish the assembled state. In short, the assembled state is permanent and very stable. The advantage of a reversible connection between the coupling portion and the handheld device is that either one can be easily and quickly disposed of in case it has experienced damage (e.g. during use in difficult environments).

Moreover, in a preferred embodiment, parts of the spray deposition system, preferably the syringe attachment device and/or handheld device, are capable of being sterilized or the whole spray deposition system is capable of being sterilized. This capability is preferably understood as a tolerance to ethylene oxide gas without any functional damage to the spray deposition system or parts thereof. The advantage is that the whole spray deposition system may be (repeatedly) sterilized without any loss in function.

Optionally, the spray deposition system does not comprise any electric and electronic components. In this case, the spray deposition system may be operated by mechanical force (manual force) only.

In a preferred embodiment, the spray deposition system or parts thereof (e.g. the syringe attachment device and parts thereof and/or the a handheld device and parts thereof) are sterile, particularly because they have been sterilized by ethylene oxide gas sterilization.

Furthermore, the inventive spray deposition system for use in treating a living being is suggested, characterized in that the syringe channel includes a syringe which contains a solution comprising an active substance which is suitable for treating a living being.

Said use may be for treating a wounded area of skin on a living being, characterized in that the solution comprises an active substance which is suitable for treating a wounded area of skin on a living being.

The active substance may comprise or consist-of biological cells.

With reference to the following Figures, the subject according to the invention is intended to be explained in more detail without wishing to restrict said subject to the specific embodiments shown here.

Explanation to the Figures: Numbers close to a line having one arrow at each end are no reference signs, but actually indicate a length in millimeters (mm) between the two arrows. The same is true for numbers which are followed by the letter "R", wherein in this case, the length in millimeters refers to the length of a radius of a circle in millimeters (mm). Numbers close to a curve and/or numbers followed by a degree sign (°) do not indicate a length, but actually an angle. All other numbers are reference signs. A list of the reference signs is given below.

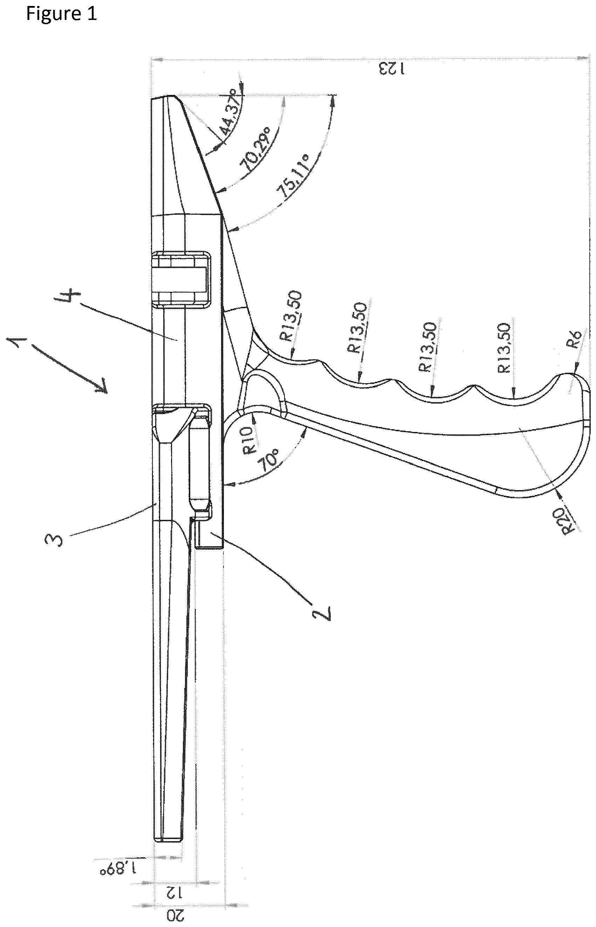

Figure 1 shows a sideview of a syringe attachment device 1 of a spray deposition system according to the invention. The syringe attachment portion 1 has a base with a coupling portion 2 and a top portion 3 and comprises a syringe channel4.

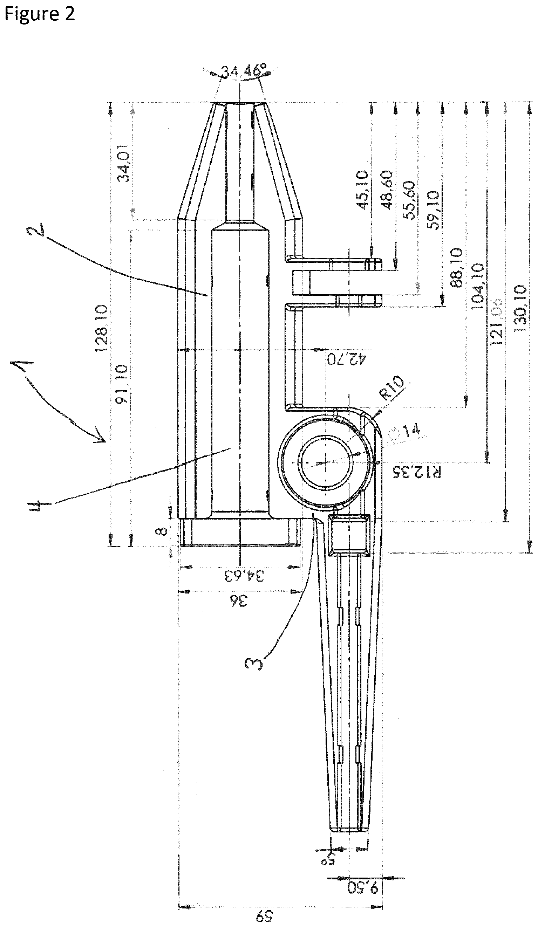

Figure 2 shows s topview of a syringe attachment device 1 of a spray deposition system according to the invention. The base with the coupling portion 2 can be seen as well as the top portion 3 and the syringe channel 4.

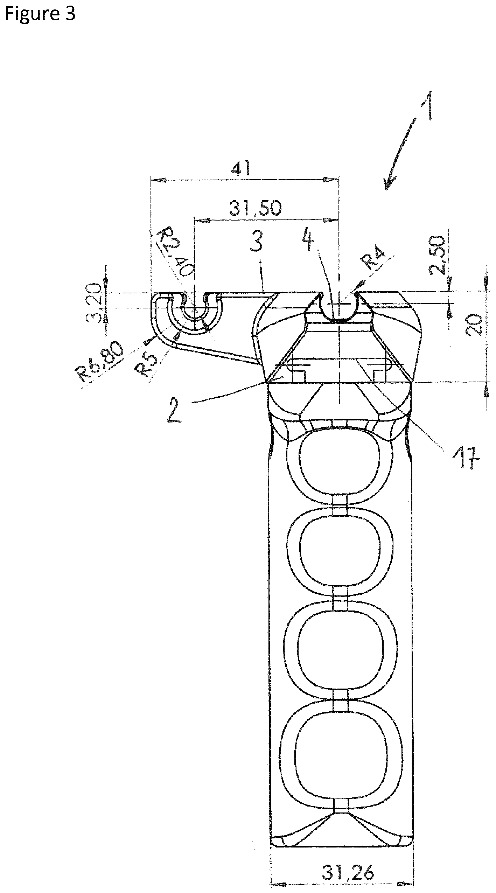

Figure 3 shows a frontview of a syringe attachment device 1 of a spray deposition system according to the invention. The base with a coupling portion 2 can be seen as well as the top portion 3 and the syringe channel 4. Additionally, the bottom surface 17 of the coupling portion 2 is illustrated.

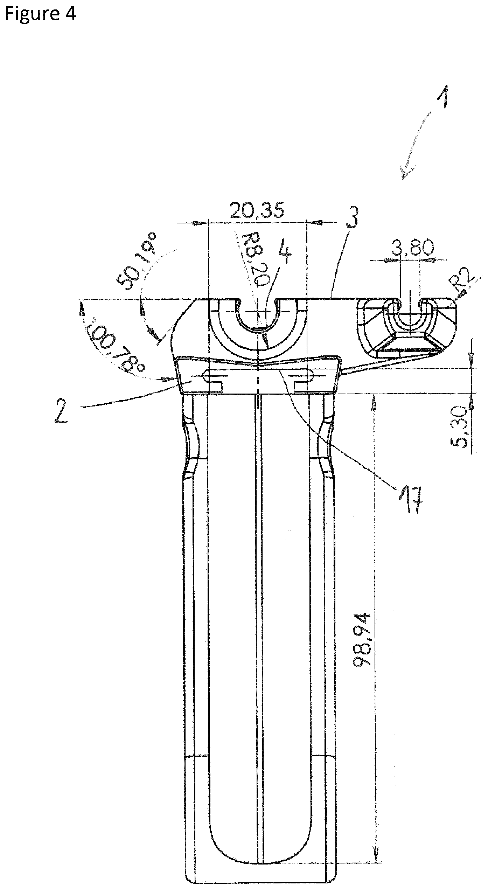

Figure 4 shows a backview of a syringe attachment device 1 of a spray deposition system according to the invention. Not only the base with a coupling portion 2, the top portion 3 and the syringe channel 4, but also the bottom surface 17 of the coupling portion 2 can be seen.

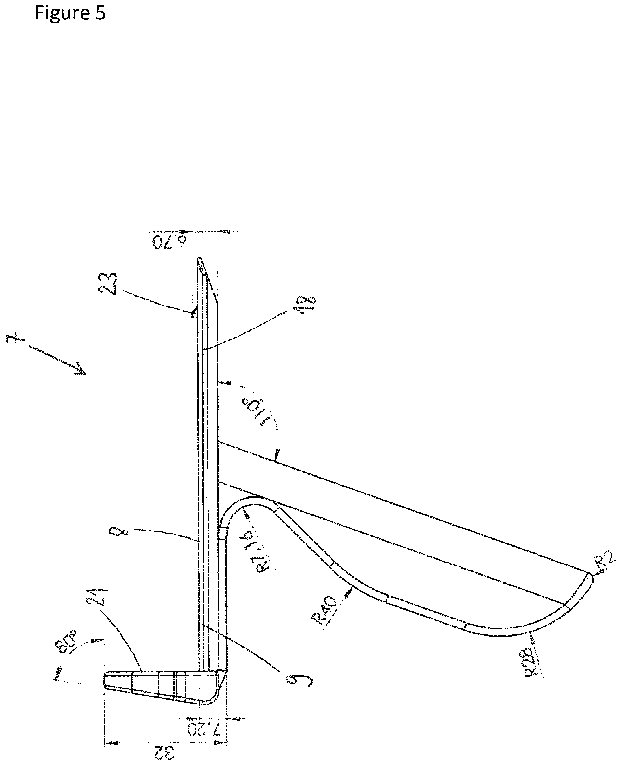

Figure 5 shows a sideview of a handheld device 7 of a spray deposition system according to the invention. The handheld device 7 has a top portion 18 with an upper surface 8 and comprises a moveable slide 9, a plunger driver 21 and a feature 23 for engaging the coupling portion of the syringe attachment device.

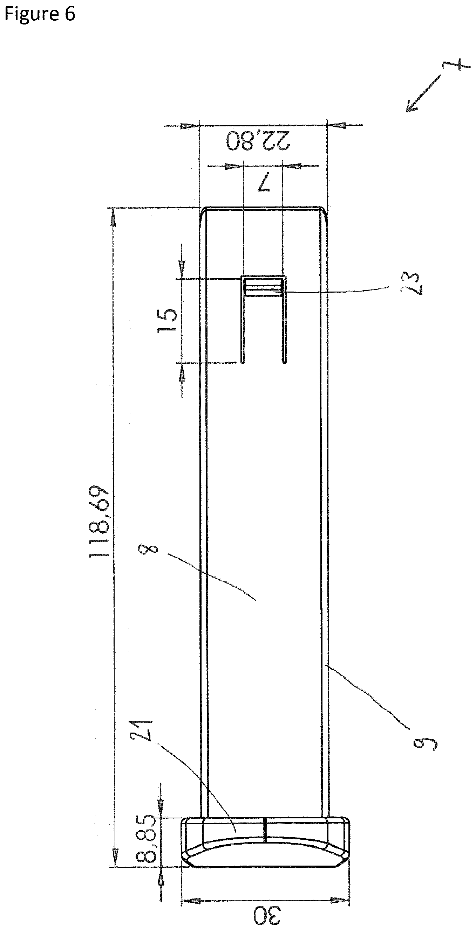

Figure 6 shows a topview of a handheld device 7 of a spray deposition system according to the invention. The moveable slide 9 and the upper surface 8 with the feature 23 for engaging the coupling portion of the syringe attachment device can be seen. Additionally, the topview of the plunger driver 21 can be seen.

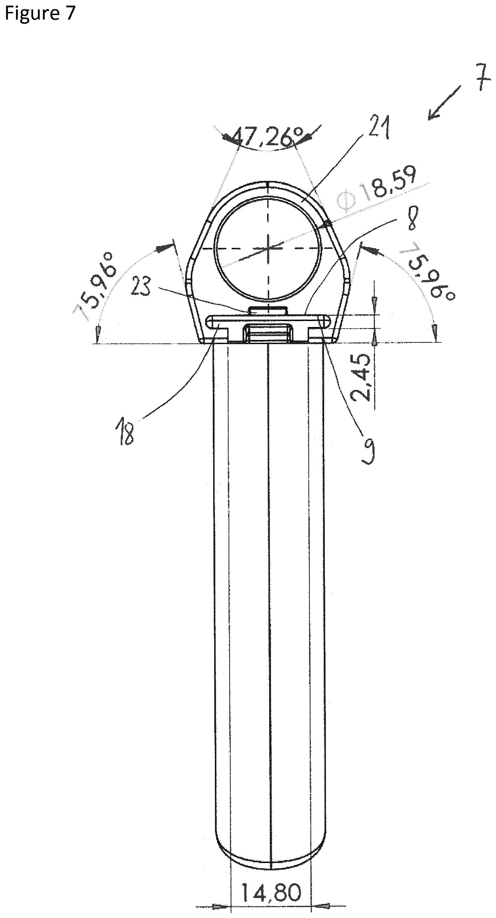

Figure 7 shows a frontview of a handheld device 7 of a spray deposition system according to the invention. On the top portion 18 of the handheld device 7, the moveable slide 9 and the upper surface 8 with the feature 23 for engaging the coupling portion of the syringe attachment device is illustrated. Additionally, the frontview of the plunger driver 21 is shown.

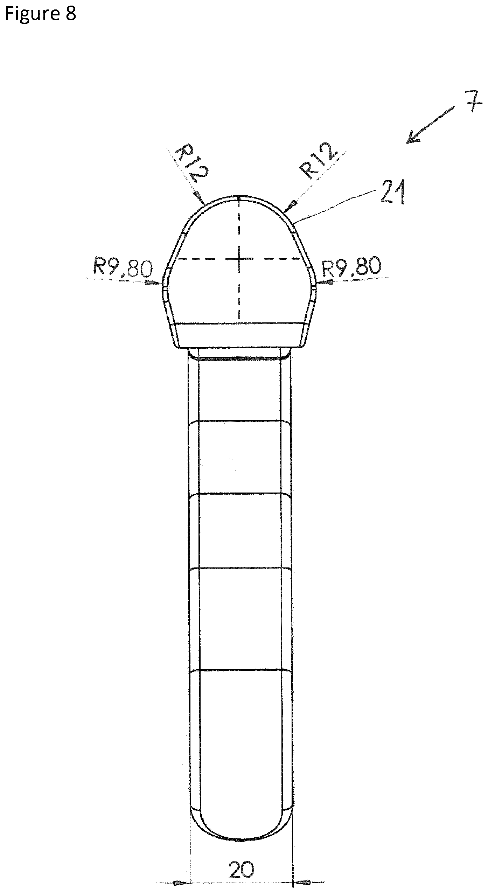

Figure 8 shows a backview of a handheld device 7 of a spray deposition system according to the invention. The backview of the plunger driver 21 is illustrated.

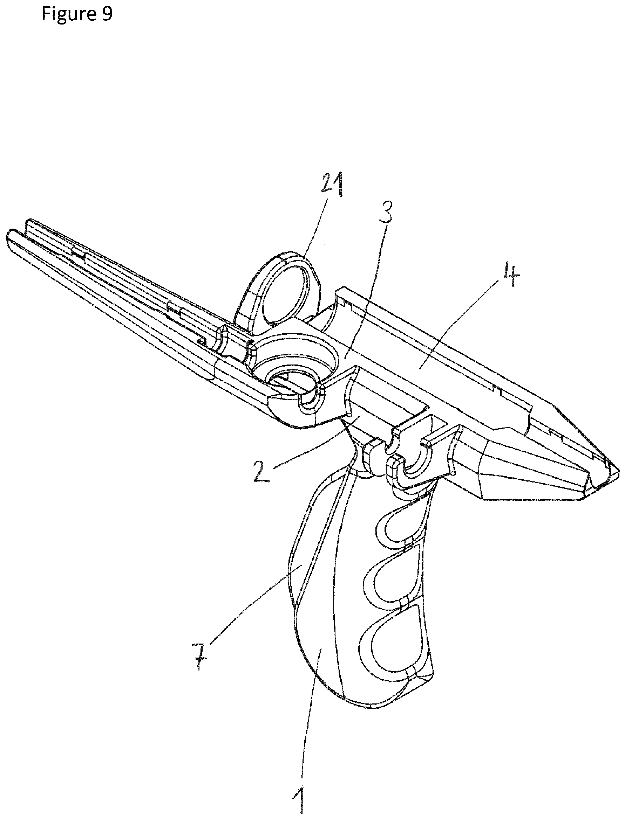

Figure 9 shows an assembled position of the spray deposition system according to the invention. The syringe attachment device 1 with its top portion 3 and syringe channel 4 is coupled to the handheld device 7 via its coupling portion 2. The coupled handheld device 7 and its plunger driver 21 are illustrated.

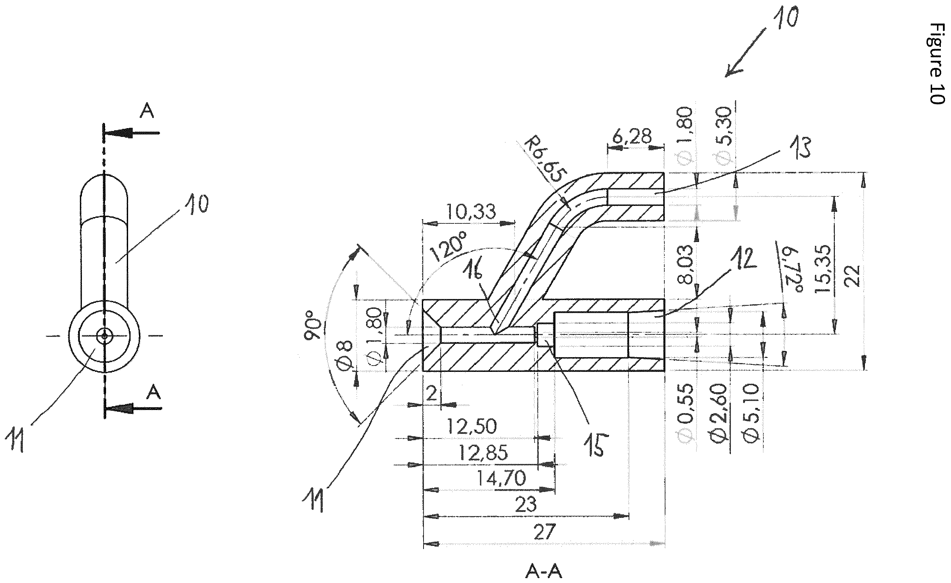

Figure 10 shows a frontview (left) and a sideview (right) of an adaptor 10 of the spray deposition system according to the invention. The first opening 11, the second opening 12 and the third opening 13 of the adaptor 10 are shown. Additionally, the first fluid channel 15 and the second fluid channel 16 of the adaptor 10 are indicated.

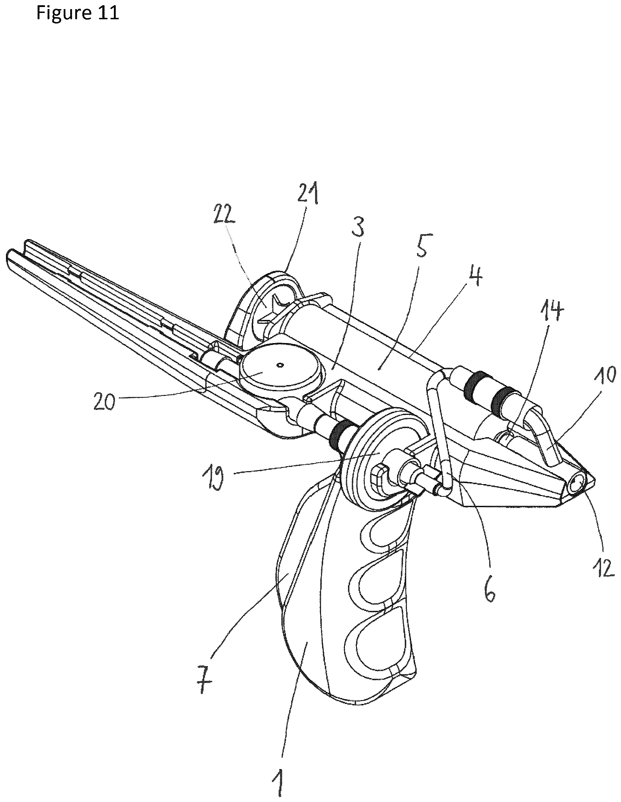

Figure 11 shows an assembled position of the spray deposition system according to the invention. The syringe attachment device 1 with its top portion 3 and syringe channel 4 is coupled to the handheld device 7 via its coupling portion. The coupled handheld device 7 and its plunger driver 21 are illustrated. The plunger driver 21 is engaged with a plunger 22 of a syringe 5. One end of a needle 14 is connected to the syringe 5 and the other end of the needle 14 is connected to the first opening of the adaptor 10. Further illustrated is the second opening 12 of the adaptor 10 which is in fluid connection with the surrounding environment (air). The third opening of the adaptor 10 is connected to a tube 6 which is configured to deliver an airflow to a first fluid channel of the adaptor 10. The tube 6 is fluidly coupled to an air filter 19 and an airflow indicator 20.

### List of reference signs:

- 1:: syringe attachment device;
- 2:: coupling portion of syringe attachment device;
- 3:: top portion of syringe attachment device;
- 4:: syringe channel of syringe attachment device;
- 5:: syringe;
- 6:: tube configured to deliver an airflow;
- 7:: handheld device;
- 8:: upper surface of handheld device;
- 9:: movable slide of handheld device;
- 10:: adaptor of syringe attachment device;
- 11:: first opening of adaptor;
- 12:: second opening of adaptor;
- 13:: third opening of adaptor;
- 14:: needle;
- 15:: first fluid channel;
- 16:: second fluid channel;
- 17:: bottom surface of coupling portion of syringe attachment device;
- 18:: top portion of handheld device;
- 19:: air filter (coupled to the tube configured to deliver an airflow);
- 20:: airflow indicator (coupled to the tube configured to deliver an airflow);
- 21:: plunger driver of movable slide of handheld device;
- 22:: plunger of a syringe;
- 23:: feature of handheld device for engaging coupling portion.

## Claims

1. A spray deposition system, comprising:
a) a syringe attachment device having a base with a coupling portion and a top portion, the syringe attachment device comprising:
i) a syringe channel configured to receive at least parts of a syringe; and
ii) a tube configured to deliver an air flow;
b) a handheld device having an upper surface and including a movable slide disposed on the upper surface, wherein the handheld device is configured to be coupled to the coupling portion of the syringe attachment device in an assembled position of the system and wherein the handheld device is configured to control delivery of a treatment solution from the syringe;
**characterized in that** the syringe attachment device comprises an adaptor having at least three openings being in fluid connection with each other, wherein at least one first opening of the adaptor is connectable to a needle in a liquid-tight manner, at least one second opening of the adaptor is in fluid connection with surrounding air and at least one third opening of the adaptor is connected to the tube in a reversible and air-tight manner.

2. The spray deposition system according to claim 1, **characterized in that** the at least one first and at least one second opening of the adaptor are fluidly connected by a first fluid channel, wherein the first fluid channel preferably has
i) an essentially straight form; and/or
ii) a length sufficiently large to accommodate a needle, preferably in a manner that the tip of the needle becomes located essentially at the same pitch like the at least one third opening; and/or
iii) a length of 20 to 40 mm, preferably 22 to 36 mm, more preferably 24 to 32 mm, most preferably 25 to 29 mm, especially 26 to 28 mm; and/or
iv) a first section for liquid-tightly, optionally also reversibly, connecting a needle adaptor suitable for connecting a needle to a syringe, wherein the first section extends from the at least one first opening of the adaptor towards the at least one second opening of the adaptor, wherein the first section optionally has a diameter of 0.5 to 8 mm, preferably 1 to 7 mm, more preferably 2 to 6 mm, most preferably 2.5 to 5.5 mm, and/or a length of 10 to 20 mm, preferably 12 to 18 mm, more preferably 14 to 16 mm; and/or
v) a second section for accommodating a needle, wherein the second section is located away from the at least one first opening of the adaptor, wherein the second section optionally has a diameter of 0.2 to 3 mm, preferably 0.5 to 2.5 mm, more preferably 1.0 to 2.0 mm, most preferably 1.4 to 1.6 mm, and/or a length of 5 to 15 mm, preferably 6 to 14, more preferably 8 to 12, most preferably 9 to 11 mm; and/or
vi) a third section for spray deposition of contents of a syringe, wherein the third section is located at the at least one second opening, wherein the third section preferably has a conical form, optionally with a maximum diameter of at least 2 mm, preferably at least 3 mm, more preferably at least 4 mm, most preferably at least 5 mm, and/or a length of 0.5 to 4 mm, preferably 1 to 2 mm, more preferably 1.5 to 2.5 mm; and/or
vii) a needle located therein, preferably a needle having a dulled tip.

3. The spray deposition system according to the preceding claims, **characterized in that** the at least one third opening is fluidly connected to the at least one first and second opening of the adaptor by a second fluid channel,, wherein the second fluid channel preferably has
i) an essentially straight form; and/or
ii) a length of 4 to 40 mm, preferably 6 to 28 mm, more preferably 8 to 16 mm, most preferably 10 to 14 mm, especially 11 to 13 mm; and/or
iii) a section for accommodating the tube configured to deliver an air flow, wherein said section is located at the at least one third opening, wherein said section preferably has a diameter of 1 to 5 mm, preferably 1.5 to 4.5 mm, more preferably 2 to 4 mm, most preferably 2.5 to 3.5 mm, and/or a shorter length than the second fluid channel, optionally a length of 1 to 30 mm, preferably 2 to 25 mm, more preferably 4 to 20 mm, most preferably 6 to 15 mm, especially 8 to 10 mm.

4. The spray deposition system according to one of the preceding claims, **characterized in that** the at least one first and at least one second opening of the adaptor are fluidly connected by a first fluid channel and the at least one third opening of the adaptor is fluidly connected to the at least one first and second opening by a second fluid channel, wherein
i) the second fluid channel fluidly connects essentially perpendicular to the first fluid channel, wherein the connection point preferably has the form of a T-shape; and/or
ii) the second fluid channel fluidly connects to the first fluid channel at a section of the first fluid channel at which a part of the needle becomes located which is closer an adaptor of the needle than to a tip of the needle.

5. The spray deposition system according to one of the preceding claims, **characterized in that** the first opening of the adaptor is suitable for reversibly and liquid-tightly connecting to a Luer-lock connector or is connected thereto, preferably a Luer-lock connector of a needle, more preferably a Luer-lock connector of a PrecisionGlide Needle 30G1/2 from Becton & Dickinson.

6. The spray deposition system according to one of the preceding claims, **characterized in that** the at least one first opening of the adaptor is connected to a needle.

7. The spray deposition system according to one of the preceding claims, **characterized in that** the adaptor
i) is sterile;
ii) comprises or consists of a plastics material, preferably a plastics material selected from the group consisting of polyurethane, polycarbonate, polyvinylchloride, acrylonitrile-butadiene-styrene, more preferably polyurethane SG 95, optionally chemically bonded with cyclohexanone and/or Loctite 4305.

8. The spray deposition system according to one of the preceding claims, **characterized in that** the syringe attachment device comprises
i) a coupling portion including a bottom surface configured to couple with a top portion of the handheld device in the assembled portion; and/or
ii) at least one feature on the coupling portion to engage a corresponding feature on an upper surface of the handheld device; and/or
iii) an air filter coupled to the tube; and/or
iv) an airflow indicator coupled to the tube; and/or
v) a needle having a taper fitting.

9. The spray deposition system according to one of the preceding claims, **characterized in that** the handheld device comprises
i) a movable slide including a plunger driver configured to engage a plunger of a syringe disposed in the syringe channel, wherein the plunger is preferably configured to be electronically positionable or manually positionable by a user of the spray deposition system; and/or
ii) a movable slide being controlled by a threaded shaft, wherein a coupler is preferably positioned between and configured for engagement with the movable slide and the threaded shaft, and movement of the coupler on the threaded shaft preferably corresponds to movement of the slide on the handheld device; and/or
iii) a switch configured to electronically control a movable slide; and/or
iv) a battery compartment, wherein an electrical contact of the battery compartment is preferably electrically coupled to the electronically movable slide; and/or
v) spray volume indicia; and/or
vi) a first mark and a sterile cover includes a second mark, and the first and second marks are configured for alignment to determine placement of the sterile cover around at least a portion of the handheld device, in the assembled position.

10. The spray deposition system according to one of the preceding claims, **characterized in that** the handheld device
i) is inserted into a sterile cover and the sterile cover encloses the handheld device in the assembled position; and/or
ii) is reusable and the syringe attachment device is disposable after use.

11. The spray deposition system according to one of the preceding claims, **characterized in that** the spray deposition system further comprises a sterile cover, wherein the sterile cover is preferably
i) coupled to the syringe attachment device, optionally between a top plate and a bottom plate of the syringe attachment device; and/or
ii) coupled to the syringe attachment device, wherein a top portion of the syringe attachment device is exposed and the sterile cover surrounds the coupling portion of the syringe attachment device; and/or
iii) welded to an outer perimeter of the base of the syringe attachment device; and/or
iv) bonded with an adhesive to an outer perimeter of the base of the syringe attachment device.

12. The spray deposition system according to one of the preceding claims, **characterized in that** the spray deposition system further comprises a sterile cover, wherein the sterile cover
i) has a cavity in which the handheld device is at least partially located in the assembled position; and/or
ii) encloses the handheld device in the assembled position; and/or
iii) has a first end which is closed and a second end which is open, wherein the second end is preferably sealable after the handheld device is inserted into a sterile cover; and/or
iv) is a sheet open on all sides, wherein the handheld device is covered by the sheet in an assembled position, and the sheet can be closed such that a seal is created between an interior of the sterile cover, containing the handheld device, and exterior of the sterile cover.

13. The spray deposition system according to one of the preceding claims, **characterized in that** the coupling portion of the syringe attachment device is coupled to the handheld device in a reversible manner or in an irreversible manner, wherein preferably, the coupling portion of the syringe attachment device is monolithic to the handheld device.

14. The spray deposition system according to one of the preceding claims, **characterized in that** parts of the spray deposition system, preferably the syringe attachment device and/or handheld device, are capable of being sterilized or the whole spray deposition system is capable of being sterilized, wherein this capability is preferably understood as a tolerance to ethylene oxide gas without any functional damage to the spray deposition system or parts thereof.

15. The spray deposition system according to one of the preceding claims for use in treating a living being, **characterized in that** the syringe channel includes a syringe which contains a solution comprising an active substance which is suitable for treating a living being, preferably for treating a wounded area of skin on a living being, and wherein the active substance preferably comprises or consists of biological cells.
